# EUROPEAN PATENT APPLICATION

(11) **EP 4 834 880 A1**
(43) Date of publication of application: **04.11.2026**
(21) Application number: 24911285.5
(22) Date of filing: 26.12.2024
(51) Int. Cl.: C07D 211/34, C07C 67/36

(54) **METHOD FOR PREPARING PHENYLPIPERIDINE DERIVATIVE**

(30) Priority: 27.12.2023 CN 202311814865
(71) Applicant: Haisco Pharmaceutical Group Co., Ltd., Shannan, Xizang 856099 (CN)
(72) Inventor: CHENG, Xunguan, Shannan, Xizang 856000 (CN); FAN, Jiang, Shannan, Xizang 856000 (CN); LIU, Sanyong, Shannan, Xizang 856000 (CN); KE, Donglei, Shannan, Xizang 856000 (CN); LI, Xintao, Shannan, Xizang 856000 (CN); ZHAO, Dayuan, Shannan, Xizang 856000 (CN)
(74) Representative: AWA Denmark A/S
(86) International application number: PCT/CN2024/142580
(87) International publication number: WO 2025/140363

(57) **Abstract**

Provided in the present invention is a method for preparing a phenylpiperidine derivative (formula (I)), where starting materials are cheap and readily available, a reaction route is simple and convenient, operation is simple, intermediates in various steps have high yields, and the method is suitable for industrial production.

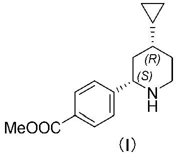

## Description

### Technical Field

The present invention relates to a method for preparing a pharmaceutical compound, and in particular to a method for preparing a phenylpiperidine derivative, which belongs to the technical field of pharmaceutical chemistry.

### Background Art

Complement factor B is a component of the complement alternative pathway and participates in the body's specific and non-specific immune mechanisms. It contains a serine protease (SP) domain. When activated, it will provide catalytic activity of C3 and C5 convertases of the alternative pathway. Complement factor B circulates as an inactive proenzyme *(i.e.,* zymogen) and is activated only after being cleaved by protein factor D. However, protein factor D can only cleave complement factor B when bound to C3:C3 (H2O) and C3b in the activated form. Complement factor B is produced as a single-chain protein and is cleaved by factor D to produce two peptide fragments (Ba and Bb). The Bb region (containing the SP domain) remains bound to C3 (H2O) and C3b, forming the alternative pathway convertase |C3(H2O)Bb and C3bBb]. The SP domain of Bb, as part of the C3 convertase, has special catalytic activity for the cleavage of C3 molecules. Adding another C3b molecule to C3 convertase of the alternative pathway can produce C5 convertase (C3bBbC3b). As part of the C5 convertase of the alternative pathway, the SP domain of Bb cleaves the C5 molecule, allowing C5-C9 to assemble and ultimately form the membrane attack complex (MAC), which participates in mediating various kidney diseases through cell lysis, promoting the release of cytokines and inflammatory mediators, cooperating with cytokines and promoting collagen IV synthesis. Therefore, complement factor B is a key enzyme in the activation process of the complement alternative pathway and can be used as a suitable target to inhibit the complement activation pathway.

Patent WO 2023020566 recites a compound of formula (A), which has good inhibitory activity against complement factor B (CFB). A compound of formula (I) is an intermediate for the synthesis of the compound of formula (A):

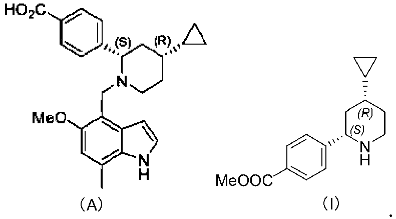

The synthetic route for the compound of formula (I) described in the above-mentioned patent is as follows:

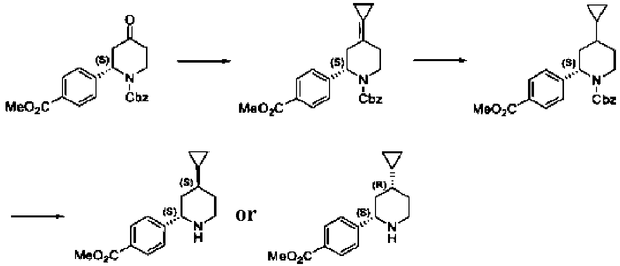

In the above-mentioned preparation method, the starting materials are expensive and difficult to obtain, and the products from each step require purification by silica gel column chromatography, resulting in low efficiency, poor yield, and high costs, making it unsuitable for industrial scale-up.

### Summary of the Invention

The present invention provides a method for preparing a compound of formula (I), comprising the following reaction:

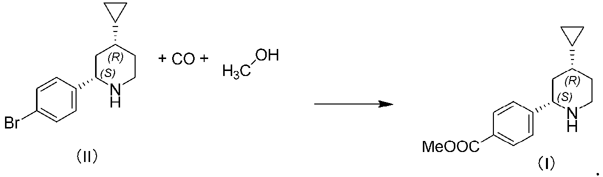

In some embodiments, the reaction is carried out in the presence of a catalyst selected from DPPF palladium dichloride, DPPP palladium acetate, allylpalladium chloride dimer, or Pd2(dba)3, preferably DPPF palladium dichloride or DPPP palladium acetate.

In some embodiments, the reaction is carried out in the presence of a base selected from triethylamine, N,N-diisopropylethylamine, or triisopropylamine, preferably triethylamine or N,N-diisopropylethylamine.

In some embodiments, the CO has a pressure of 0.1-3.0 Mpa, preferably 0.5-0.8 Mpa.

In some embodiments, the reaction is carried out in the presence of a solvent, wherein the solvent is methanol.

In some embodiments, the reaction temperature of the reaction is 40°C-120°C, preferably 60°C±5°C.

The present invention further provides a method for refining the compound of formula (I), comprising: 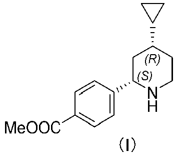
1) dissolving a crude compound of formula (I) in an organic solvent;
2) adding an organic acid for salt formation and crystallisation; and
3) filtering the solution to obtain a refined compound of formula (I).

In some embodiments, the organic solvent is selected from at least one of isopropyl acetate, ethyl acetate, and methyl tert-butyl ether, preferably isopropyl acetate or ethyl acetate; the organic acid is selected from at least one of naphthalenedisulphonic acid, succinic acid, tartaric acid, and maleic acid, preferably naphthalenedisulphonic acid.

The present invention further provides a method for preparing a compound of formula (II), comprising the following reaction:

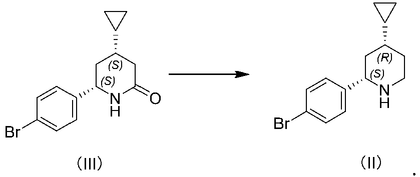

In some embodiments, the reaction is carried out in the presence of a reducing agent selected from at least one of sodium borohydride, lithium borohydride, potassium borohydride, and borane, preferably sodium borohydride.

In some embodiments, the reaction is carried out in the presence of a catalyst selected from at least one of boron trifluoride tetrahydrofuran complex, boron trifluoride diethyl ether complex, calcium chloride, lithium chloride, and iodine, preferably boron trifluoride tetrahydrofuran complex or boron trifluoride diethyl ether complex.

In some embodiments, the reaction is carried out in the presence of a solvent selected from at least one of tetrahydrofuran, diethyl ether, and methyltetrahydrofuran, preferably tetrahydrofuran.

In some embodiments, the reaction temperature of the reaction is 30°C-70°C, preferably 55°C±5°C.

The present invention further provides a method for preparing a compound of formula (III), comprising the following reaction:

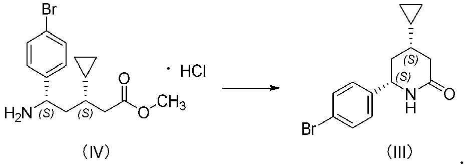

In some embodiments, the reaction is carried out in the presence of a base selected from at least one of triethylamine, N,N-diisopropylethylamine, triisopropylamine, potassium carbonate, sodium carbonate, sodium hydroxide, and potassium hydroxide, preferably triethylamine or N,N-diisopropylethylamine.

In some embodiments, the feeding molar ratio of the compound of formula (IV) to the base is 1 : 2-6, preferably 1 : 2.5-4.0.

In some embodiments, the reaction temperature of the reaction is 20°C-100°C, preferably 80°C±5°C.

The present invention further provides a method for preparing a compound of formula (IV), comprising the following reaction: 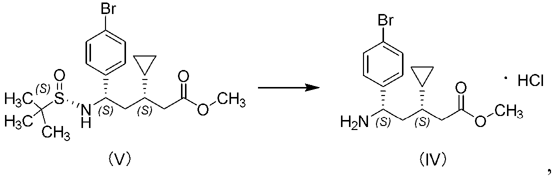 wherein the reaction is carried out in the presence of hydrogen chloride, the hydrogen chloride is added in the form of a solution in an organic solvent, and the organic solvent is selected from at least one of methanol, ethanol, and isopropanol, preferably methanol or isopropanol.

In some embodiments, the reaction temperature of the reaction is 0°C-80°C, preferably 45°C±5°C.

The present invention further provides a method for preparing a compound of formula (V), comprising the following reaction:

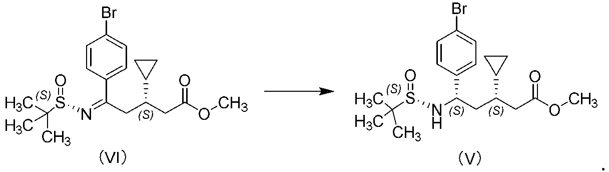

In some embodiments, the reaction is carried out in the presence of a catalyst and a catalyst ligand, wherein the catalyst is selected from dichloro(p-cymene)ruthenium(II) dimer or dichlorotris(triphenylphosphine)ruthenium, and the catalyst ligand is selected from 2-amino-2-methyl-1-propanol or 2-amino-2-ethyl-1-propanol.

In some embodiments, the reaction is carried out in the presence of a solvent selected from isopropanol or 2-butanol.

In some embodiments, the reaction temperature of the reaction is 30°C-80°C, preferably 45°C±5°C.

The present invention further provides a method for preparing a compound of formula (VI), comprising the following reaction:

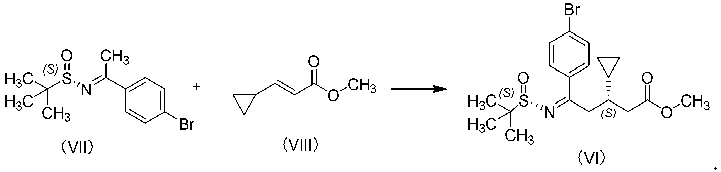

In some embodiments, the reaction is carried out in the presence of a catalyst selected from at least one of lithium diisopropylamide, lithium diethylamide, and n-butyllithium, preferably lithium diisopropylamide.

In some embodiments, the reaction is carried out in the presence of a solvent selected from methyltetrahydrofuran or tetrahydrofuran.

In some embodiments, the reaction temperature of the reaction is -70°C-10°C, preferably -25°C±5°C.

The present invention further provides a method for preparing a compound of formula (VII), comprising the following reaction:

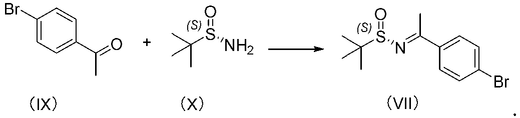

In some embodiments, the reaction is carried out in the presence of a catalyst selected from tetraethyl titanate or tetraisopropyl titanate.

In some embodiments, the reaction temperature of the reaction is 40°C-120°C, preferably 60°C±5°C.

In addition, the present invention further provides another method for preparing a compound of formula (I), comprising the following reaction:

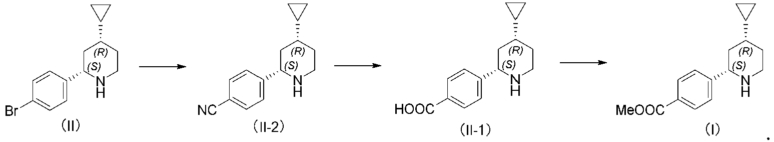

In some embodiments, formula (II) is reacted in the presence of a complex catalyst to obtain formula (II-2), wherein the complex catalyst is selected from allylpalladium(II) chloride dimer/2-dicyclohexylphosphino-2,6-diisopropoxy-1,1-biphenyl, or bis(triphenylphosphine)palladium dichloride/2-dicyclohexylphosphino-2,6-diisopropoxy-1,1-biphenyl.

In some embodiments, formula (II) is reacted in the presence of potassium hexacyanoferrate(II) or a hydrate thereof, or zinc cyanide, cuprous cyanide, potassium cyanide, or sodium cyanide to obtain formula (II-2), preferably in the presence of potassium hexacyanoferrate(II) trihydrate.

In some embodiments, formula (II) is reacted in the presence of a base to obtain formula (II-2), wherein the base is selected from at least one of N,N-diisopropylethylamine, triethylamine, tripropylamine, and triisopropylamine, preferably N,N-diisopropylethylamine or triethylamine.

In some embodiments, the reaction solvent for the conversion of formula (II) to formula (II-2) is selected from at least one of an aqueous mixture of 2-methyl-2-butanol and water, an aqueous mixture of tert-butanol and water, and an aqueous mixture of tetrahydrofuran and water, preferably an aqueous mixture of 2-methyl-2-butanol and water or an aqueous mixture of tert-butanol and water.

In some embodiments, the reaction temperature for the conversion of formula (II) to formula (II-2) is 60°C-100°C, preferably 90°C±5°C.

In some embodiments, the formula (II-2) is reacted in the presence of a base to obtain formula (II-1), wherein the base is selected from at least one of sodium hydroxide, potassium hydroxide, and lithium hydroxide, preferably sodium hydroxide or potassium hydroxide.

In some embodiments, the reaction solvent for the conversion of formula (II-2) to formula (II-1) is selected from at least one of an aqueous mixture of methanol and water, an aqueous mixture of ethanol and water, and an aqueous mixture of tetrahydrofuran and water, preferably an aqueous mixture of methanol and water or an aqueous mixture of ethanol and water.

In some embodiments, the reaction temperature for the conversion of formula (II-2) to formula (II-1) is 50°C-80°C, preferably 75°C±5°C.

In some embodiments, the formula (II-1) is reacted in the presence of methanol and thionyl chloride or an acid to obtain formula (I), wherein the acid is selected from at least one of hydrogen chloride, hydrogen bromide, and sulphuric acid, preferably hydrogen chloride or sulphuric acid.

In some embodiments, the reaction solvent for the conversion of formula (II-1) to formula (I) is methanol.

In some embodiments, the reaction temperature for the conversion of formula (II-1) to formula (I) is 20°C-80°C, preferably 70°C±5°C.

The methods for preparing the above-mentioned compounds of the present invention are not limited to obtaining a certain compound from a single reaction step as described above, but also include steps for preparing intermediates of the compound. For example, the method for preparing the compound of formula (VI) is not limited to the step of reacting the compound of formula (VII) with the compound of formula (VIII) to obtain the compound of formula (VI), but may also include the step of preparing the compound of formula (VII) from the compound of formula (IX). The reaction conditions for each step are consistent with those of the corresponding steps described previously.

For example,
the present invention further provides a method for preparing a compound of formula (VI), comprising the following steps: 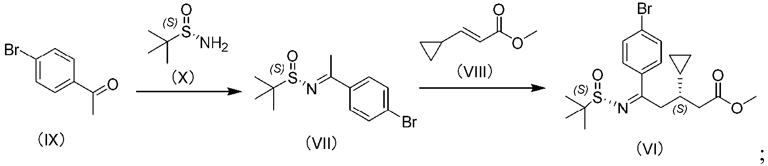
the present invention further provides a method for preparing a compound of formula (V), comprising the following steps: 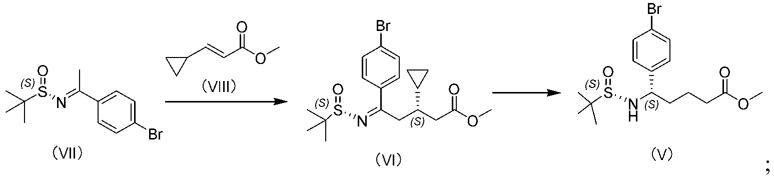
further, the method for preparing the compound of formula (V) of the present invention comprises the following steps: 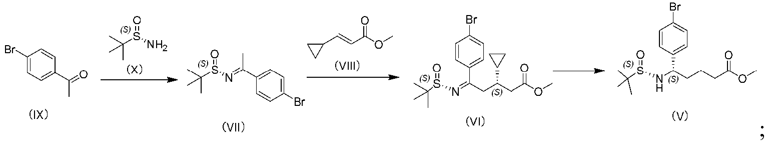
**similarly,** the method for preparing the compound of formula (IV) of the present invention comprises the steps of (VI)→(V)→(IV), and may also comprise the steps of (VII)→(VI)→(V)→(IV), or (IX)→(VII)→(VI)→(V)→(IV);
similarly, the method for preparing the compound of formula (III) of the present invention comprises the steps of (V)→(IV)→(III), and may also comprise the steps of (VI)→(V)→(IV)→(III), or (VII)→(VI)→(V)→(IV)→(III), or (IX)→(VII)→(VI)→(V)→(IV)→(III);
similarly, the method for preparing the compound of formula (II) of the present invention comprises the steps of (IV)→(III)→(II), and may also comprise the steps of (V)→(IV)→(III)→(II), or (VI)→(V)→(IV)→(III)→(II), or (VII)→(VI)→(V)→(IV)→(III)→(II), or (IX)→(VII)→(VI)→(V)→(IV)→(III)→(II);
similarly, the method for preparing the compound of formula (I) of the present invention comprises the steps of (III)→(II)→(I), and may also comprise the steps of (IV)→(III)→(II)→(I), or (V)→(IV)→(III)→(II)→(I), or (VI)→(V)→(IV)→(III)→(II)→(I), or (VII)→(VI)→(V)→(IV)→(III)→(II)→(I);
in particular, the present invention further provides a method for preparing a compound of formula (I), comprising the following steps:

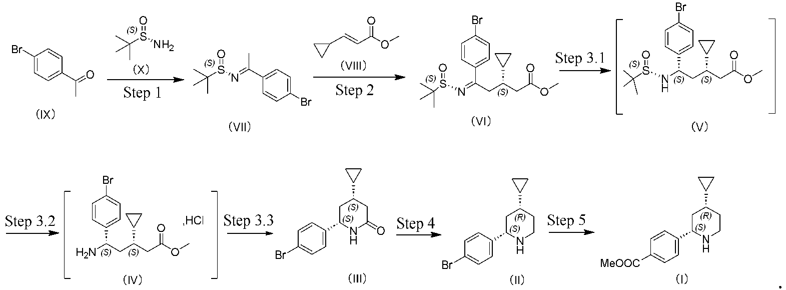

In some embodiments, the step 1 is carried out in the presence of a catalyst selected from tetraethyl titanate or tetraisopropyl titanate;
in some embodiments, the step 2 is carried out in the presence of a catalyst selected from at least one of lithium diisopropylamide and lithium diethylamide, preferably lithium diisopropylamide;
in some embodiments, the step 3.1 is carried out in the presence of a catalyst and a catalyst ligand, wherein the catalyst is selected from dichloro(p-cymene)ruthenium(II) dimer or dichlorotris(triphenylphosphine)ruthenium, and the catalyst ligand is selected from 2-amino-2-methyl-1-propanol or 2-amino-2-ethyl-1-propanol;
in some embodiments, the step 3.2 is carried out in the presence of hydrogen chloride/an organic solvent, wherein the organic solvent is selected from at least one of methanol, ethanol, and isopropanol, preferably methanol or isopropanol;
in some embodiments, the step 3.3 is carried out in the presence of a base selected from at least one of triethylamine, N,N-diisopropylethylamine, triisopropylamine, potassium carbonate, sodium carbonate, sodium hydroxide, and potassium hydroxide, preferably triethylamine or N,N-diisopropylethylamine;
in some embodiments, in the step 3.3, the feeding molar ratio of the compound of formula (IV) to the base is 1 : 2-6, preferably 1 : 2.5-4.0;
in some embodiments, the step 4 is carried out in the presence of a reducing agent selected from at least one of sodium borohydride, lithium borohydride, potassium borohydride, and borane, preferably sodium borohydride;
in some embodiments, the step 4 is carried out in the presence of a catalyst selected from at least one of boron trifluoride tetrahydrofuran complex, boron trifluoride diethyl ether complex, calcium chloride, lithium chloride, and iodine, preferably boron trifluoride tetrahydrofuran complex or boron trifluoride diethyl ether complex;
in some embodiments, the step 5 is carried out in the presence of a catalyst selected from DPPF palladium dichloride, DPPP palladium acetate, allylpalladium chloride dimer, or tris(dibenzylideneacetone)dipalladium, preferably DPPF palladium dichloride or DPPP palladium acetate;
in some embodiments, the step 5 is carried out in the presence of a base selected from triethylamine, N,N-diisopropylethylamine, or triisopropylamine, preferably triethylamine or N,N-diisopropylethylamine.

In addition, the present invention further provides a method for preparing a compound of formula (I), comprising the following steps:

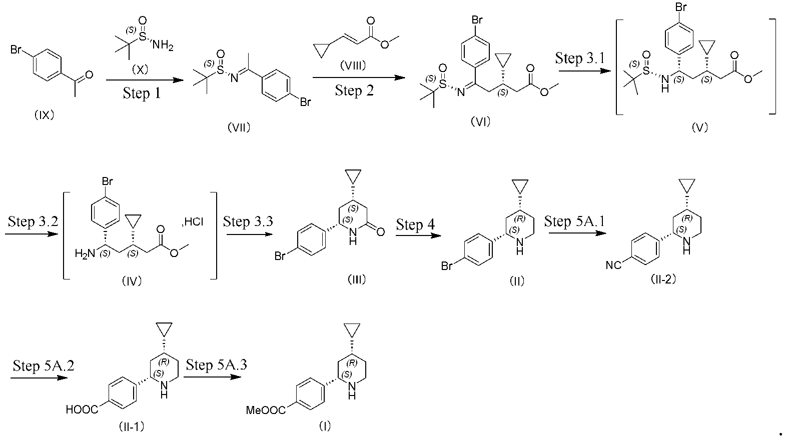

In some embodiments, the step 1 is carried out in the presence of a catalyst selected from tetraethyl titanate or tetraisopropyl titanate;
in some embodiments, the step 2 is carried out in the presence of a catalyst selected from at least one of lithium diisopropylamide and lithium diethylamide, preferably lithium diisopropylamide;
in some embodiments, the step 3.1 is carried out in the presence of a catalyst and a catalyst ligand, wherein the catalyst is selected from dichloro(p-cymene)ruthenium(II) dimer or dichlorotris(triphenylphosphine)ruthenium, and the catalyst ligand is selected from 2-amino-2-methyl-1-propanol or 2-amino-2-ethyl-1-propanol;
in some embodiments, the step 3.2 is carried out in the presence of hydrogen chloride/an organic solvent, wherein the organic solvent is selected from at least one of methanol, ethanol, and isopropanol, preferably methanol or isopropanol;
in some embodiments, the step 3.3 is carried out in the presence of a base selected from at least one of triethylamine, N,N-diisopropylethylamine, triisopropylamine, potassium carbonate, sodium carbonate, sodium hydroxide, and potassium hydroxide, preferably triethylamine or N,N-diisopropylethylamine;
in some embodiments, in the step 3.3, the feeding molar ratio of the compound of formula (IV) to the base is 1 : 2-6, preferably 1 : 2.5-4.0;
in some embodiments, the step 4 is carried out in the presence of a reducing agent selected from at least one of sodium borohydride, lithium borohydride, potassium borohydride, and borane, preferably sodium borohydride;
in some embodiments, the step 4 is carried out in the presence of a catalyst selected from at least one of boron trifluoride tetrahydrofuran complex, boron trifluoride diethyl ether complex, calcium chloride, lithium chloride, and iodine, preferably boron trifluoride tetrahydrofuran complex or boron trifluoride diethyl ether complex;
in some embodiments, the step 5A.1 is carried out in the presence of a complex catalyst selected from allylpalladium(II) chloride dimer/2-dicyclohexylphosphino-2,6-diisopropoxy-1,1-biphenyl, or bis(triphenylphosphine)palladium dichloride/2-dicyclohexylphosphino-2,6-diisopropoxy-1,1-biphenyl;
in some embodiments, the step 5A.1 is carried out in the presence of potassium hexacyanoferrate(II) or a hydrate thereof, or zinc cyanide, cuprous cyanide, potassium cyanide, or sodium cyanide, preferably in the presence of potassium hexacyanoferrate(II) trihydrate;
in some embodiments, the step 5A.1 is carried out in the presence of a base selected from at least one of N,N-diisopropylethylamine, triethylamine, tripropylamine, and triisopropylamine, preferably N,N-diisopropylethylamine;
in some embodiments, the step 5A.2 is carried out in the presence of a base selected from at least one of sodium hydroxide, potassium hydroxide, and lithium hydroxide, preferably sodium hydroxide or potassium hydroxide;
in some embodiments, the step 5A.3 is carried out in the presence of methanol and thionyl chloride or an acid, wherein the acid is selected from at least one of hydrogen chloride, hydrogen bromide, and sulphuric acid, preferably hydrogen chloride or sulphuric acid.

The present invention further provides a compound of formula (II), (II-1), (II-2), (III), (IV), (V), or (VI), or a salt thereof:

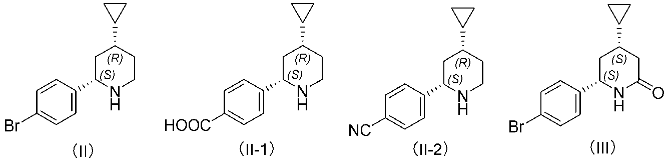

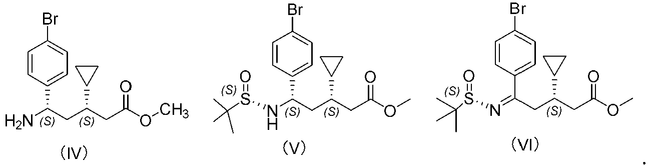

Definitions of abbreviations and key terms in the present invention:

| Abbreviation or term | English name | Abbreviation or term | English name |
|---|---|---|---|
| Ti(OEt)4 | Tetraethyl titanate | MeOH | Methanol |
| MeTHF | 2-Methyltetrahydrofuran | TEA | Triethylamine |
| RuCl₂ | Ruthenium dichloride | DPPF | 1,1'-Bis(diphenylphosphino) ferrocene |
| 1-iPr | 1-Isopropyl | LDA | Lithium diisopropylamide |
| 4-MePh- | 4-Methylphenyl | TLC | Thin-layer chromatography |
| AMP | 2-Amino-2-methyl-1-propanol | [Pd(C3H5)Cl]2 | Allylpalladium(II) chloride dimer |
| Pd2(dba)3 | Tris(dibenzylideneacetone)d ipalladium | Ruphos | 2-Dicyclohexylphosphino -2,6-diisopropoxy-1,1-biphenyl |
| RuCl2(PPh3)3 | Dichlorotris(triphenylphosp hine)ruthenium | K4[Fe(CN)6].3 H2O | Potassium hexacyanoferrate(II) trihydrate |
| t-BuOK | Potassium tert-butoxide | DIPEA | N,N-Diisopropylethylamine |
| i-PrOH | Isopropanol | | |

The technical effects of the present invention are as follows.
1. The process route for synthesising formula (I) of the present invention is novel, which features mild reaction conditions, simple operation and high yield in each step, as well as high product purity and convenient workup, making it suitable for industrial production.
2. The process of the present invention has the advantages of easily available starting materials, simple steps, that is, no silica gel column chromatography or other preparative chromatography methods are used throughout the entire synthesis process, low costs, good intermediate stability, high purity and high yield, making it suitable for large-scale industrial production.

### Detailed Description of Embodiments

The present invention is further described in detail below in conjunction with examples which do not limit the present invention. Any equivalent substitutions made according to the disclosure of the present invention fall within the scope of protection of the present invention.

The structures of the compounds are determined by nuclear magnetic resonance (NMR) or (and) mass spectrometry (MS). The NMR shift (δ) is given in the unit of 10⁻⁶ (ppm). NMR is determined with NMR instruments (Bruker Avance III 400 and Bruker Avance 300); the solvent for determination is deuterated dimethylsulphoxide (DMSO-d₆), deuterated chloroform (CDCl₃) or deuterated methanol (CD₃OD); and the internal standard is tetramethylsilane (TMS).

MS is determined with Agilent 6120B (ESI) and Agilent 6120B (APCI).

HPLC is determined with Agilent 1260DAD high-performance liquid chromatograph (chromatographic column: Zorbax SB-C18 100×4.6 mm, 3.5 µm).

For thin-layer chromatography (TLC), silica gel plates from Yantai Huanghai HSGF254 or Qingdao GF254 are used. Silica gel plates with a thickness of 0.15 mm-0.20 mm are employed for TLC, while plates with a thickness of 0.4 mm-0.5 mm are used for TLC separation and purification.

### Example 1

### (S,E)-N-(1-(4-bromophenyl)ethylidene)-2-methylpropane-2-sulfinamide (compound of formula (VII))

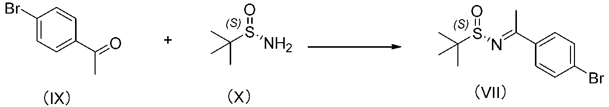

Under a nitrogen atmosphere, 1.00 kg of the compound of formula (IX), 0.913 kg of the compound of formula (X), and 4.01 kg of tetraethyl titanate were added to a reactor, and the mixture was heated to 60°C and allowed to react. After the reaction was completed, 8 kg of ethyl acetate and 15 kg of purified water were added. The mixture was stirred and filtered, and the filtrate was collected. The filter cake was washed with ethyl acetate. The filtrates were combined and subjected to phase separation. The organic phase was collected and washed with 20% sodium chloride solution. The phase separation was conducted, and the organic phase was collected. The organic phase was concentrated to dryness under reduced pressure below 50°C. 4 kg of n-heptane was added, and the mixture was stirred to induce crystallisation, filtered, and dried in vacuum to obtain 1.20 kg of the intermediate compound of formula (VII). HPLC purity: 99.00%, yield: 80%.

### Example 2

### methyl (S,Z)-5-(4-bromophenyl)-5-(((S)-tert-butylsulfinyl)imino)-3-cyclopropylpentanoate (compound of formula (VI))

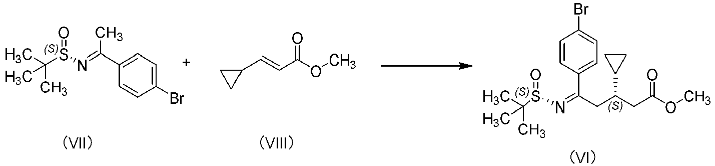

A reactor was purged with nitrogen, and 0.50 kg of the compound of formula (VII), 0.522 kg of the compound of formula (VIII), and 4.0 kg of 2-methyltetrahydrofuran were added to the reactor under a nitrogen atmosphere. After the addition was completed, the mixture was cooled to -25°C±5°C. 1.08 L of LDA solution was added dropwise with the temperature controlled at -25°C±5°C. After the dropwise addition was completed, the mixture was reacted for 2.5 h with the temperature maintained.

After the reaction was completed, an aqueous acetic acid solution (0.130 kg of acetic acid dissolved in 1 kg of water) was added dropwise with the temperature controlled below 0°C. After the dropwise addition was completed, the mixture was stirred and left to stand for phase separation. The organic phase was collected. The organic phase was washed with 20% aqueous sodium chloride solution.

The organic phase was concentrated to dryness under reduced pressure below 50°C. 1.2 kg of n-hexane was added, and the mixture was cooled to -10°C±5°C and stirred to induce crystallisation. After crystallisation was completed, the system was filtered. The filter cake was collected and dried, and the residue was collected to obtain 0.40 kg of the intermediate compound of formula (VI).

HPLC purity: 97.00%, yield: 55%.

### Example 3

### (4S,6S)-6-(4-bromophenyl)-4-cyclopropylpiperidin-2-one (compound of formula (III))

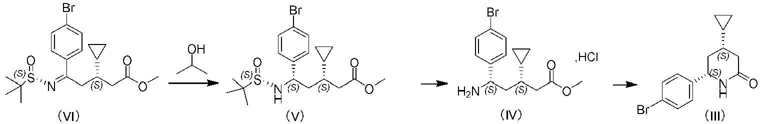

Under a nitrogen atmosphere, 0.8 kg of isopropanol, 10.72 g of dichloro(p-cymene)ruthenium(II) dimer, and 3.12 g of 2-amino-2-methyl-1-propanol (AMP) were added. The mixture was heated to reflux for about 0.5 h, and then cooled to 45°C±5°C to obtain a pre-prepared ligand solution for later use.

Under a nitrogen atmosphere, 300 g of the compound of formula (VI) and 2.4 kg of isopropanol were heated to 45°C±5°C with stirring until dissolved. After purging with nitrogen for 1 h, the pre-prepared ligand solution and 15.72 g of potassium tert-butoxide were added. After the addition was completed, the mixture was reacted for about 1 h with the temperature controlled at 45°C±5°C. The reaction was monitored by TLC until the compound of formula (VI) was completely consumed, affording the compound of formula (V).

After the reaction was completed, 0.7 L of hydrogen chloride/methanol solution (4 M) was added to the reaction solution, and the mixture was reacted at 45°C±5°C for about 1 h. The reaction was monitored by TLC until the reaction was completed, affording the compound of formula (IV).

After the reaction was completed, 400 g of triethylamine was added, and the mixture was reacted for about 2 h with the temperature controlled at 80°C±5°C. The reaction was monitored by TLC until the reaction was completed.

After the reaction was completed, the system was concentrated under reduced pressure at 50°C±5°C to remove most of the solvent. 1.8 kg of purified water was added, and the mixture was cooled to 25°C±5°C, stirred to induce crystallisation, filtered, and washed with water, and the filter cake was collected. The filter cake was added to methyl tert-butyl ether, and the mixture was heated to 50°C±5°C and stirred. n-Heptane was added, and the mixture was cooled to 10°C±5°C and stirred to induce crystallisation.

After crystallisation was completed, the system was filtered, and the filter cake was washed with n-heptane. The filter cake was dried under reduced pressure, and the residue was collected to obtain 120 g of the intermediate compound of formula (III).

HPLC purity: 98.00%, yield: 65%.

### Example 4

### (2S,4R)-2-(4-bromophenyl)-4-cyclopropylpiperidine (compound of formula (II))

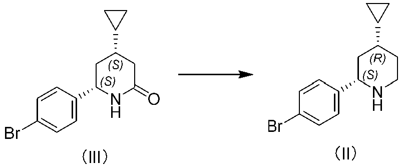

0.30 kg of the compound of formula (III) and 2.7 kg of tetrahydrofuran were stirred until dissolved. 0.097 kg of sodium borohydride was added, and then 0.428 kg of boron trifluoride tetrahydrofuran complex was added dropwise. The mixture was heated to 55°C±5°C and maintained at this temperature until the reaction was completed.

4.5 kg of purified water was added to another reactor, and the above reduction reaction solution was slowly added under stirring. After the addition was completed, the mixture was stirred. Hydrochloric acid was added to adjust the pH to 1-2. The mixture was heated to 55°C±5°C and maintained at this temperature for the reaction.

After the reaction was completed, the system was cooled to below 30°C. Solid sodium hydroxide was added to adjust the pH to 8-9, and the mixture was stirred. The mixture was extracted with 0.45 kg of isopropyl acetate. The phase separation was conducted, and the organic phase was collected. The aqueous phase was extracted with isopropyl acetate. The phase separation was conducted, and the organic phase was collected.

The organic phase was washed with 20% aqueous sodium chloride solution, and the phase separation was conducted. The organic phase was concentrated to dryness under reduced pressure at 50°C±5°C to obtain 0.275 kg of the intermediate compound of formula (II).

HPLC purity: 96.00%, yield: 90%.

### Example 5

### methyl 4-((2S,4R)-4-cyclopropylpiperidin-2-yl)benzoate (compound of formula (I))

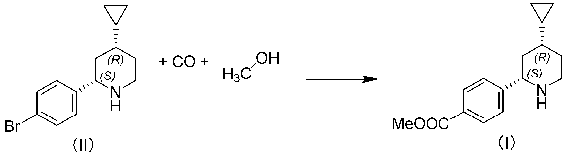

0.15 kg of the compound of formula (II), 1.2 kg of methanol, 108.3 g of triethylamine, and 11.8 g of DPPF palladium dichloride were added. The system was purged with CO, and then CO was introduced to a pressure of 0.5-0.8 Mpa. The system was heated to 60°C±5°C and reacted until completion.

After the reaction was completed, the system was filtered through a pad of diatomaceous earth. The filter cake was rinsed with methanol. The filtrates were combined and concentrated to dryness under reduced pressure at 50°C±5°C. 1.5 kg of isopropyl acetate was added, and the mixture was heated to 60°C±5°C until completely dissolved. The resulting solution was washed twice with 0.3 kg of cysteine/potassium phosphate aqueous solution (prepared by dissolving 0.05 kg of cysteine and 0.05 kg of potassium phosphate in 0.2 kg of purified water with stirring for later use). The phase separation was conducted. The organic phase was collected and washed with 20% aqueous sodium chloride solution. The organic phase was collected and concentrated to dryness under reduced pressure at 50°C±5°C to obtain 0.132 kg of the crude compound of formula (I).

0.132 kg of the crude compound of formula (I) was added to 1.0 kg of isopropyl acetate, and the mixture was heated to 50°C±5°C until dissolved. A solution of 150 g of naphthalenedisulphonic acid in 0.5 kg of isopropanol was added. The mixture was stirred for 2 h and then cooled to room temperature to induce crystallisation. The mixture was filtered, and the filter cake was added to 1 L of sodium hydroxide solution (1 mol/L) and stirred until dissolved. The system was extracted twice with isopropyl acetate. The organic phase was collected and washed with 20% aqueous sodium chloride solution. The organic phase was collected and concentrated to dryness under reduced pressure at 50°C±5°C to obtain 0.118 kg of the compound of formula (I).

HPLC purity: 98.49%, chiral purity: 98.5%, yield: 85.0%.

### Example 6

### 4-((2S,4R)-4-cyclopropylpiperidin-2-yl)benzonitrile (compound of formula (II-2))

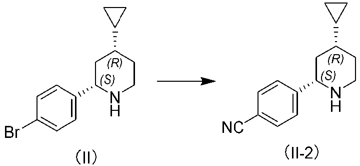

1.00 kg of the compound of formula (II), 0.61 kg of N,N-diisopropylethylamine, 0.40 kg of potassium hexacyanoferrate(II) trihydrate, 4.85 kg of 2-methyl-2-butanol, and 8 kg of purified water were added to a reactor, and the mixture was purged with N₂ for more than 1 hour.

In another reactor, 4.3 g of allylpalladium(II) chloride dimer and 22 g of 2-dicyclohexylphosphino-2,6-diisopropoxy-1,1-biphenyl (Ruphos) were added to 100 g of 2-methyl-2-butanol and 100 g of purified water, and the mixture was purged with N₂ for more than 1 hour.

The complexed catalyst was added to the reactor, and the mixture was heated to 90°C±5°C and reacted for about 18 h.

After the reaction was completed, the system was cooled, and the phase separation was conducted. The aqueous phase was extracted with isopropyl acetate, and the organic phases were combined. The organic phase was washed with 20% sodium chloride solution, and the organic phase was collected. The organic phase was concentrated under reduced pressure at 60°C±5°C to remove 2-methyl-2-butanol. 2 kg of anhydrous ethanol was added, and the mixture was subjected to azeotropic distillation until a large amount of solid precipitated. 10 kg of purified water was added, and the mixture was stirred for 1 hour. The mixture was filtered, and the filter cake was dried under reduced pressure at 55°C±5°C. The residue was collected to obtain 804 g of the intermediate compound of formula (II-2).

HPLC purity: 98.10%, yield: 92%.

### Example 7

### 4-((2S,4R)-4-cyclopropylpiperidin-2-yl)benzoic acid (compound of formula (II-1))

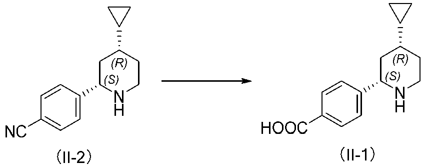

1 kg of the compound of formula (II-2) and 5 kg of methanol were stirred, and 0.94 kg of sodium hydroxide and 5 kg of purified water were added under stirring. The mixture was heated to 80°C±5°C and reacted for more than 18 h.

After the reaction was completed, the system was concentrated under reduced pressure to remove most of methanol with the temperature controlled at 60°C±5°C. After concentration was completed, the system was cooled to below 20°C and adjusted to pH 5-6 with hydrochloric acid, and then a large amount of solid precipitated. The mixture was stirred with the temperature controlled at 20°C±5°C. The mixture was filtered, and the filter cake was washed with isopropanol. The filter cake was dried under reduced pressure at 60°C±5°C to obtain 965 g of the intermediate compound of formula (II-1).

HPLC purity: 98.10%, yield: 90.4%.

### Example 8

### methyl 4-((2S,4R)-4-cyclopropylpiperidin-2-yl)benzoate (compound of formula (I))

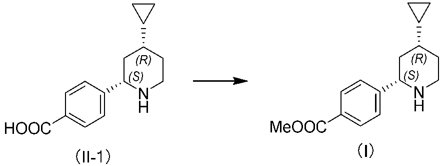

### Subexample 1:

1 kg of the compound of formula (II-1), 8 kg of methanol, and a solution of hydrogen chloride in methanol (0.61 L, 4 mol/L) were added, and the mixture was heated to 70°C±5°C and reacted for about 8 h. After the reaction was completed, the system was concentrated at 60°C±5°C to remove most of methanol. 5 kg of purified water was added, and the mixture was adjusted to pH 7-8 with 6 M sodium hydroxide solution. The mixture was extracted with 3 kg of isopropyl acetate. The phase separation was conducted, and the aqueous phase was further extracted with isopropyl acetate. The phase separation was conducted, and the organic phase was collected.

5.9 kg of cysteine/potassium phosphate aqueous solution (prepared by dissolving 0.2 kg of cysteine and 0.7 kg of potassium phosphate in 5 kg of purified water with stirring for later use) was added to the organic phase. The mixture was heated to 60°C±5°C and stirred for 30 min. The phase separation was conducted, and the organic phase was collected and washed with 20% aqueous sodium chloride solution. The organic phase was concentrated to dryness under reduced pressure at 50°C±5°C to obtain 986 g of the compound of formula (I).

HPLC purity: 99.10%, yield: 92.9%.

### Subexample 2:

4.00 kg of methanol and 1.00 kg of the compound of formula (II-1) were added to a reactor, and 0.73 kg of thionyl chloride was added dropwise. After the addition was completed, the mixture was heated to reflux (about 63°C±5°C) and reacted. After the reaction was completed, the mixture was cooled to 0°C±5°C. 3.00 kg of water was added with the temperature controlled at 0°C±5°C, and the mixture was stirred for 0.5 hours. The mixture was adjusted to pH 10-11 with 50% aqueous potassium carbonate solution with the temperature controlled at 5°C±5°C. 10.00 kg of water was added, and the mixture was stirred to induce crystallisation with the temperature controlled at 5°C±5°C. The resulting mixture was filtered and dried, to obtain the compound of formula (I) (yield: 95%).

## Claims

1. A method for preparing a compound of formula (I), **characterised by** comprising the following reaction:

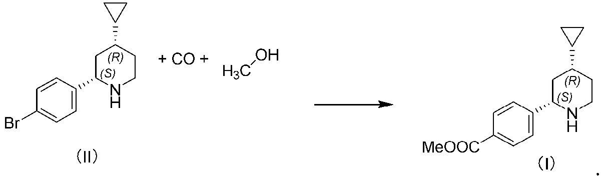

2. The preparation method according to claim 1, **characterised in that** the reaction is carried out in the presence of a catalyst selected from DPPF palladium dichloride, DPPP palladium acetate, allylpalladium chloride dimer, or tris(dibenzylideneacetone)dipalladium, preferably DPPF palladium dichloride or DPPP palladium acetate;
the reaction is carried out in the presence of a base selected from triethylamine, N,N-diisopropylethylamine, or triisopropylamine, preferably triethylamine or N,N-diisopropylethylamine.

3. A method for preparing a compound of formula (II), **characterised by** comprising the following reaction:

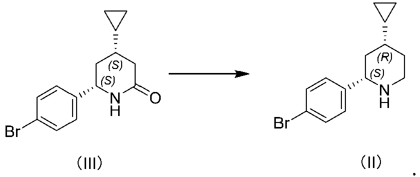

4. The preparation method according to claim 3, **characterised in that** the reaction is carried out in the presence of a reducing agent selected from at least one of sodium borohydride, lithium borohydride, potassium borohydride, and borane, preferably sodium borohydride;
the reaction is carried out in the presence of a catalyst selected from at least one of boron trifluoride tetrahydrofuran complex, boron trifluoride diethyl ether complex, calcium chloride, lithium chloride, and iodine, preferably boron trifluoride tetrahydrofuran complex or boron trifluoride diethyl ether complex.

5. A method for preparing a compound of formula (III), **characterised by** comprising the following reaction:

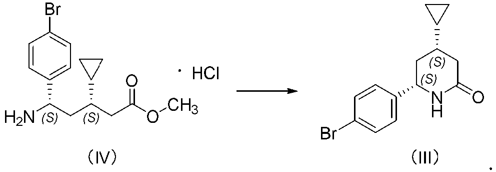

6. The preparation method according to claim 5, **characterised in that** the reaction is carried out in the presence of a base selected from at least one of triethylamine, N,N-diisopropylethylamine, triisopropylamine, potassium carbonate, sodium carbonate, sodium hydroxide, and potassium hydroxide, preferably triethylamine or N,N-diisopropylethylamine.

7. A method for preparing a compound of formula (V), **characterised by** comprising the following reaction:

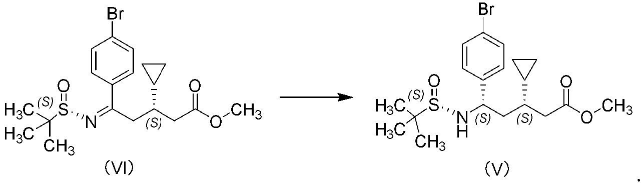

8. The preparation method according to claim 7, **characterised in that** the reaction is carried out in the presence of a catalyst and a catalyst ligand, and the catalyst is selected from dichloro(p-cymene)ruthenium(II) dimer or RuCl₂ (PPh₃)₃;
the catalyst ligand is selected from 2-amino-2-methyl-1-propanol or 2-amino-2-ethyl-1-propanol.

9. A method for preparing a compound of formula (VI), **characterised by** comprising the following reaction:

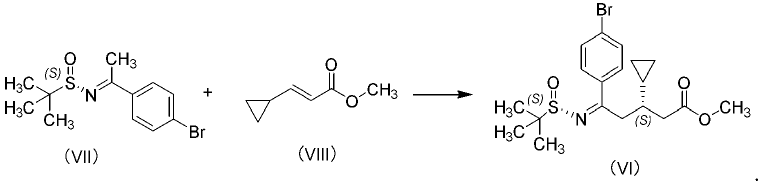

10. The preparation method according to claim 9, **characterised in that** the reaction is carried out in the presence of a catalyst selected from at least one of lithium diisopropylamide, lithium diethylamide, and n-butyllithium, preferably lithium diisopropylamide.

11. A method for preparing a compound of formula (I), **characterised by** comprising the following reaction:

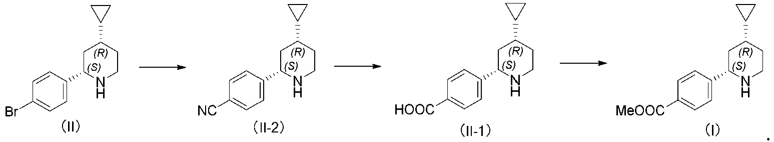

12. The preparation method according to claim 11, **characterised in that** formula (II) is reacted in the presence of a complex catalyst to obtain formula (II-2), and the complex catalyst is selected from allylpalladium(II) chloride dimer/2-dicyclohexylphosphino-2,6-diisopropoxy-1,1-biphenyl, or bis(triphenylphosphine)palladium dichloride/2-dicyclohexylphosphino-2,6-diisopropoxy-1,1-biphenyl;
formula (II) is reacted in the presence of potassium hexacyanoferrate(II) or a hydrate thereof, or zinc cyanide, cuprous cyanide, potassium cyanide, or sodium cyanide to obtain formula (II-2), preferably in the presence of potassium hexacyanoferrate(II) trihydrate;
formula (II) is reacted in the presence of a base to obtain formula (II-2), and the base is selected from at least one of N,N-diisopropylethylamine, triethylamine, tripropylamine, and triisopropylamine, preferably N,N-diisopropylethylamine or triethylamine;
the formula (II-2) is reacted in the presence of a base to obtain formula (II-1), and the base is selected from at least one of sodium hydroxide, potassium hydroxide, and lithium hydroxide, preferably sodium hydroxide or potassium hydroxide;
the formula (II-1) is reacted in the presence of methanol and thionyl chloride or an acid to obtain formula (I), and the acid is selected from at least one of hydrogen chloride, hydrogen bromide, and sulphuric acid, preferably hydrogen chloride or sulphuric acid.

13. The method for preparing the compound of formula (I) according to any one of claims 1, 2, 11 and 12, **characterised by** further comprising the following steps:

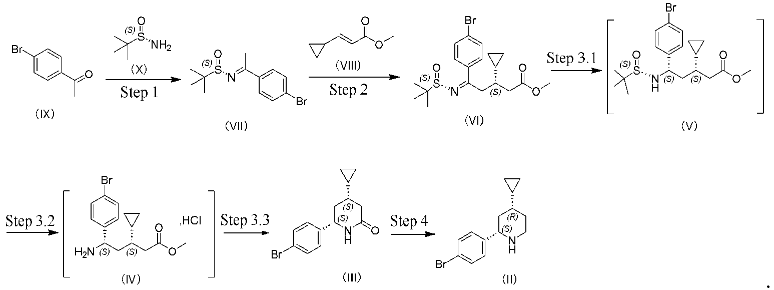

14. The preparation method according to claim 13, **characterised in that** the step 1 is carried out in the presence of a catalyst selected from tetraethyl titanate or tetraisopropyl titanate;
the step 2 is carried out in the presence of a catalyst selected from at least one of lithium diisopropylamide and lithium diethylamide, preferably lithium diisopropylamide;
the step 3.1 is carried out in the presence of a catalyst selected from dichloro(p-cymene)ruthenium(II) dimer or dichlorotris(triphenylphosphine)ruthenium;
the step 3.1 is carried out in the presence of a catalyst ligand selected from 2-amino-2-methyl-1-propanol or 2-amino-2-ethyl-1-propanol;
the step 3.2 is carried out in the presence of hydrogen chloride/an organic solvent, and the organic solvent is selected from at least one of methanol, ethanol, and isopropanol, preferably methanol or isopropanol;
the step 3.3 is carried out in the presence of a base selected from at least one of triethylamine, N,N-diisopropylethylamine, triisopropylamine, potassium carbonate, sodium carbonate, sodium hydroxide, and potassium hydroxide, preferably triethylamine or N,N-diisopropylethylamine;
the step 4 is carried out in the presence of a reducing agent selected from sodium borohydride, lithium borohydride, potassium borohydride, and borane, preferably sodium borohydride;
the step 4 is carried out in the presence of a catalyst selected from at least one of boron trifluoride tetrahydrofuran complex, boron trifluoride diethyl ether complex, calcium chloride, lithium chloride, and iodine, preferably boron trifluoride tetrahydrofuran complex or boron trifluoride diethyl ether complex.

15. A compound of formula (II), (II-1), (II-2), (III), (IV), (V), or (VI), or a salt thereof:
